# EUROPEAN PATENT SPECIFICATION

(11) **EP 0 862 930 B1**
(45) Date of publication and mention of the grant of the patent: **15.10.2003**
(21) Application number: 98103780.7
(22) Date of filing: 04.03.1998
(51) Int. Cl.: A63B 23/04, A61B 5/103

(54) **An interactive device with a balance plate**
Interaktive Vorrichtung mit einer Gleichgewichtsplatte
Appareil interactif avec plateau d'équilibrage

(30) Priority: 06.03.1997 IT TO970183
(43) Date of publication of application: 09.09.1998
(73) Proprietor: Delos S.r.l., 10138 Torino (IT); Riva Violetta, Dario, 10146 Torino (IT); Kratter, Gioachino, 10156 Torino (IT)
(72) Inventor: Riva Violetta, Dario, 10146 Torino (IT); Kratter, Gioachino, 10156 Torino (IT)
(74) Representative: Rambelli, Paolo

(56) References cited:
- WO-A-91/05587
- DE-A- 19 502 838
- FR-A- 2 641 183
- FR-A- 2 731 158

## Description

The present invention relates to an interactive device with a balance plate for the assessment and training of the equilibrium capacity and disequilibrium management and for the evaluation, training and rehabilitation of the proprioceptive sensitivity and reflexes in man.

Equilibrium (balance) is the ability of a person to maintain or to recover a certain static or dynamic position in a manner which is functional with respect to the force of gravity and appropriate for the success of the action. From the point of view of the structures involved, the term equilibrium means a form of complex co-ordination in which several factors are involved; in order of importance, these are: visual information, tactile-proprioceptive information, and vestibular information. Visual information makes the adjustment of posture and of movements more precise. It is integrated with the tactile information coming from the parts of the body in contact with a supporting surface and the proprioceptive information which indicates the magnitude of the muscle contraction and informs the central nervous system of the positions of the various body segments. The vestibular information coming from the labyrinth provides information on the position of the head and on its linear and angular accelerations.

The proprioceptive sensitivity permits neuromuscular programming and reprogramming based on the integration of the messages constantly emitted by the peripheral receptors. Pro-prioceptive rehabilitation is therefore based on information coming from the periphery and influencing the central systems controlling movement. It aims, in particular, to re-establish correct timing in the execution of complex motor activities in which the integration of information carried out at the level of the central nervous system has a determining role (for example, in maintaining balance).

At the moment, so-called "proprioceptive boards" are characterised by rocking plates (with various degrees of freedom) which are supported on the floor and are used mainly in the rehabilitation of the proprioceptive reflexes, particularly for the after-effects of joint trauma to the ankle and the knee and for the prevention thereof.

From a physiological point of view, use is made of the assumptions of a technique known as "proprioceptive neuromuscular facilitation" which enables the muscles involved to operate with regard to the following types of information:
- information of surface origin (tactile information coming from the sole of the foot supported);
- information of deep origin (joint position, stretching of the tendons, of the capsule-ligament structures and of the neuromuscular spindles);
- information of visual and vestibular origin.

EP-A-0 484 247 describes a balance plate which can be used in the rehabilitation field and which is mounted for pivoting about a horizontal axis with a fixed fulcrum disposed below the plane of the balance plate on which the user's feet are supported. This device has a transducer for transforming the angular position of the plate relative to the horizontal into an electrical signal; the signal processor comprises rectifier means for rectifying the signal and means for integrating the electrical signal produced by the rectifying means over time in order to supply an integrated value which can be displayed by a display means.

The signal generated by the apparatus and displayed on the display means does not accurately represent the instantaneous movement of the pivoting plate, since it is an integrated value.

EP-A-0 448 684 describes an interactive balance plate having a fixed fulcrum and a centre of rotation on the centreline of the plate. In this case and even more in the device of EP-A-0 484 247, the device involved is highly unstable and a disequilibrium condition results in large accelerations which are difficult to control within the average reaction time of an individual, which is about 200 milliseconds.

FR-A-2 641 183 also describes a balance plate with a fixed fulcrum.

An object of the present invention is to provide an instrument which is fully interactive with the person using it, which provides constant visual feedback relating to his equilibrium management ability so that the person is informed about the position of his foot in space in real time in order to permit a significant and measurable improvement in the aforesaid ability.

Another object of the invention is to provide a device which can measure the actual proprioceptive sensitivity of each limb in absolute and comparative manner, in the form of a test, and which can inform the person in real time about the position in space of the surface supporting his foot/feet.

The feedback, which is preferably visual but may be acoustic or of another type, helps the person to improve the ability of his central analysers to interpret the proprioceptive signals coming from the periphery and trains him to program ever more quickly and effectively his postural or dynamic response to the situation to be managed.

In view of these objects, the subject of the invention is a device with a balance plate particularly for the assessment and training of equilibrium capacity and disequilibrium management ability and for the evaluation, training and rehabilitation of the proprioceptive sensitivity and reflexes, as defined by the appended claims.

According to a first preferred embodiment A, the member for contacting the fixed support has a cylindrical, preferably circular cylindrical, contact profile. More preferably, the rocking plate has two spaced-apart contact members with circular cylindrical contact surfaces.

According to another preferred embodiment B, the contact member has a spherical contact surface.

Further characteristics and advantages of the device according to the invention will become clear from the following detailed description given with reference to the appended drawings, in which:
- Figure 1 is a schematic view of the device,
- Figure 2 is an exploded, perspective view of the balance plate,
- Figure 3 is a detail taken on the arrow III of Figure 2,
- Figure 4 is a block diagram showing signal-generating and processing devices,
- Figures 5 and 6 are perspective views of other embodiments.

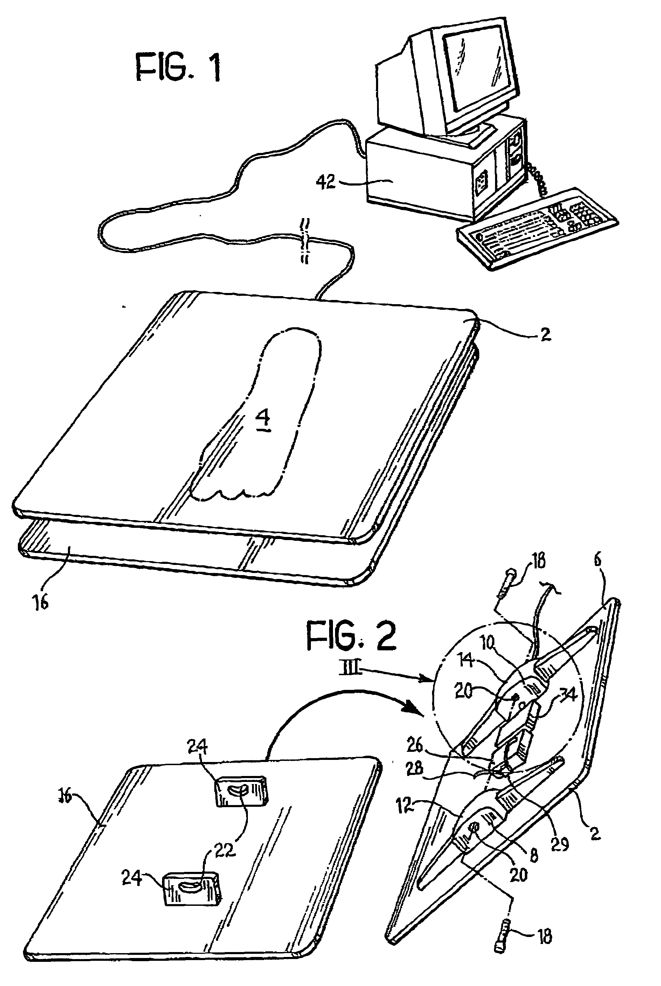

With reference to the drawings, the device according to the invention comprises a rocking plate 2 having an upper surface 4 for supporting the user's foot or feet. The plate 2 may have any appropriate shapes, such as particularly a square, rectangular or circular shape. According to embodiment A of Figures 1-3, the plate has two contact members 8 and 10, fixed to the lower face 6 of the plate 2, which are intended to bear on a fixed support and which have respective circular cylindrical contact surfaces 12 and 14.

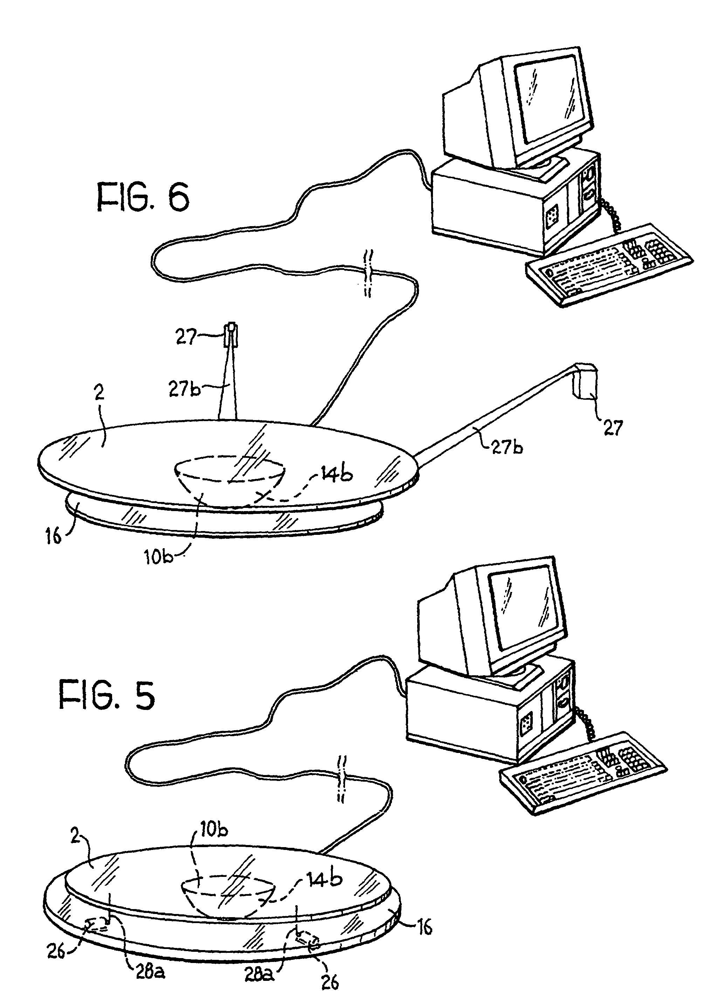

According to embodiment B, the plate 2 is provided with a contact member 10b, having a spherical contact surface 14b (Figures 5 and 6).

The radius of curvature of the contact surfaces 12, 14 and 14b is such that the centre of rotation of the rocking plate is located above the upper support surface 4. The radius of curvature is preferably between 4 and 10 cm. In the preferred configuration, the radius of curvature is 6 cm. The downward offset of the plate from the axis of rotation, meaning the distance of the support surface from the instantaneous centre of rotation, is about 1-2 cm in the preferred configuration and may be between 0.5 and 9 cm.

The rocking plate 2 may be used bearing directly on the ground or on a floor but, in the preferred embodiment A, it is connected to a fixed support plate 16 so that the contact members 8 and 10 bear on the upper face of the support. The use of the fixed plate allows repeatable conditions during different tests. Variations in hardness and friction offered by different support surfaces would render tests both of the same person and of different people scarcely comparable.

The connection between the rocking plate 2 and the fixed plate 16 is suitable to provide the necessary degree of freedom to allow rocking movements. For this purpose, the connection comprises, for each contact member 8 or 10, engagement means 18 constituted, for example, by a threaded pin inserted in a through-hole 20 in the respective contact member and fitted slidingly in a respective arcuate slot 22 formed in a flange 24 perpendicular to the fixed plate 16.

The range of the inclination angle of the rocking plate relative to the horizontal is preferably less than 20° and more preferably about 13-15°, most preferably 14°.

Although the balance plate described above comprises a pair of contact members 8 and 10, rocking plates having a single contact member formed in accordance with the invention are intended to fall within the scope of the invention.

By virtue of these characteristics, a deviation from the equilibrium position causes the instantaneous centre of rotation of the plate to be translated in space in a plane located above the upper surface of the plate 4, thus forming a rocking system with a movable fulcrum. Said instantaneous centre of rotation moves along a straight path, in case of embodiment A.

An analysis of the rocking motion of the plate reveals that the angular acceleration resulting from a displacement from the equilibrium position decreases rapidly and progressively and then becomes negative (deceleration) well before the maximum inclination angle (typically 14°), that is, the angle at which the rocking plate touches the fixed plate, is reached. By virtue of these characteristics, the system allows the whole of the travel to be utilised in conditions compatible with the person's reaction times for all the degrees of inclination of the plate. These characteristics enable the user not only to keep his balance, but himself to induce and manage disequilibrium situations by trying to track selected predefined display paths with the trace produced by the movement of the plate, as will be described in greater detail below.

The rocking plate 4 and the fixed plate may be made of any rigid material, for example, metal or plastics materials.

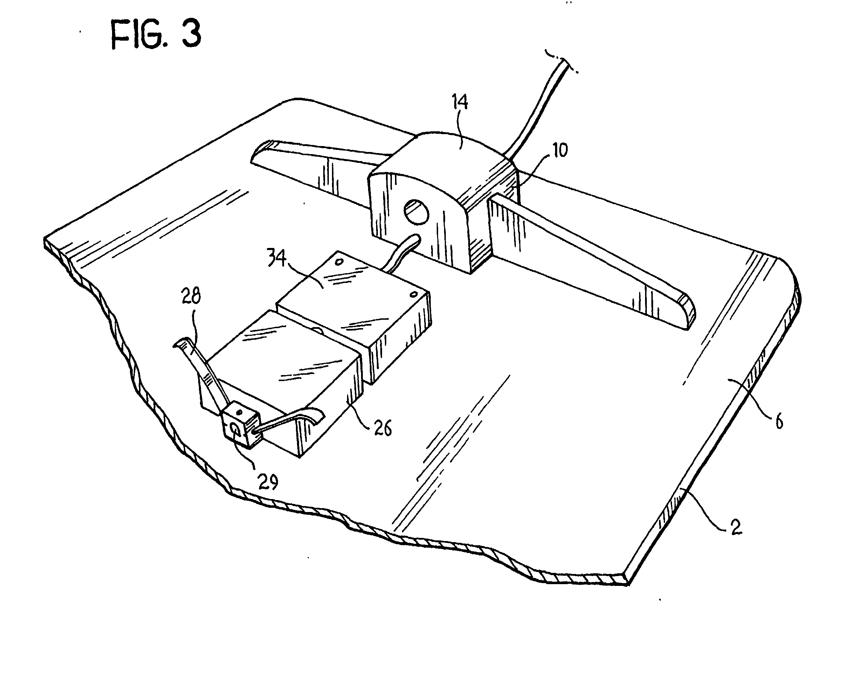

Transducer means, for example, associated with the rocking plate 2, for converting the angular position of the plate 2 relative to the horizontal into an electrical signal, are indicated 26 in Figures 2 and 3.

The angular-position transducer means may, for example, be constituted by a potentiometer or an encoder.

Since the horizontal displacement k of the generatrix of the cylinder bearing on the horizontal support surface can be related to the angle α (α being the angle of inclination of the support surface of the rocking plate to the horizontal) the angular position of the rocking plate can also be determined by means for measuring the displacement k, as well as by means for measuring α.

In the embodiment shown in Figure 2, the transducer is a potentiometer fixed to the lower face 6 of the rocking plate with a contact member 28 constituted by a spring steel plate or lamina fixed to a shaft 29 of the potentiometer.

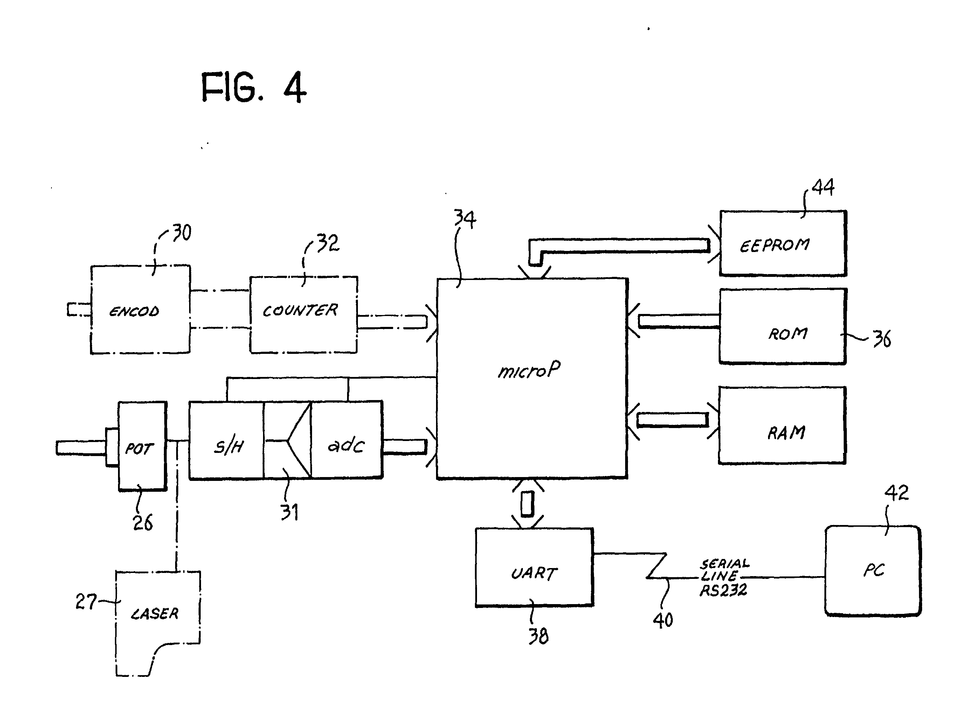

The movement of the rocking plate moves the potentiometer body around the shaft, which is not allowed to turn but only translate by the spring steel lamina 28, sliding on the fixed plate. The potentiometer rotation changes the electrical resistance measured between the movable contact and one of the two ends of the resistive element of the potentiometer. If a direct-current voltage is applied to the resistive element, a voltage related to the position of the potentiometer movable contact can be measured. A measurement of the angle of inclination of the rocking plate to the horizontal can thus be derived proportionally from the measurement of the voltage present at the movable contact by association of the potentiometer supply-voltage range with the range of movement of the platform (maximum travel). The voltage (analog signal) is acquired by means of an analog-to-digital conversion system indicated 31 in Figure 4. The analog signal is sampled and hold periodically at regular and precise intervals (with a minimum sampling period of 4 milliseconds) and each sample is converted into a number by binary encoding. This regularity of acquisition permits reliable measurement of the variations of the signal which are proportional to the speed of movement.

According to embodiment B, the angular-position measurement requires two independent transducer devices, e.g. two potentiometers 26 associated with two orthogonal axes, belonging to the plane supporting the user's foot/feet or to a plane parallel thereto.

As schematically shown in Figure 5, the plate is linked to the shaft of each potentiometer 26 by means of an extensible wire.

The signal representative of the plate inclination is obtained by combining the voltage outputs of the two transducers; the orientation in the horizontal plane defined by the alignment axes of the two transducers, can be also determined.

In Figure 4, the variant in which the transducer is constituted by an encoder 30 is shown in broken outline. In this variant, however, the electrical signals are discrete. The movement of the rocking plate causes a train of electrical pulses to be generated. The number of pulses is dependent on the displacement. These pulses have a duration and a frequency proportional to the speed of the movement. If the total number of pulses generated by the encoder within a prefixed angular range (the maximum travel) is known, a measurement of the plate inclination can therefore readily be obtained by counting the pulses. The measurement system shown in Figure 4 comprises a digital counter 32 which is read periodically at regular and precise time intervals; each count acquired is encoded into binary form.

In the case of embodiment B, the transducer means may preferably comprise, rather than the above-mentioned potentiometers or encoders, two optical laser devices 27, supported by arms 27b rigidly connected to the rocking plate 2, and aligned with two orthogonal axes; such devices are suitable for measuring the displacement without the need of mechanical contacts or of displacement transmission means. By using the "triangulation" method, said transducers provide an electrical (analog) signal which is proportional to the variation of distance between the surface of the rocking plate (foot supporting plane) and the surface of the fixed support (reference plane).

The electrical signals of each laser device are combined substantially as described in the case of the two potentiometers, used for the embodiment B .

The signal-processing is based on a microprocessor device 34, (carried by the rocking plate or externally), which selects and controls the system for acquiring the measurement of the inclination of the rocking plate. The operation of this device is controlled by specific resident firmware (stored on a Read-Only Memory 36). The microprocessor also has the task of controlling a communication device (a UART module 38) on an asynchronous serial line 40 (RS232 standard); the acquired data (the measurement of the inclination in encoded digital form) is transmitted by means of this line to an external processor 42 (PC platform) upon a specific request thereby.

The microprocessor also accesses serially (for reading/writing) a non-volatile memory 44 (an electrically-erasable programmable Read-Only Memory) used to record some set-up parameters processed by the firmware.

The PC has complete control of the interface, transmitting specific requests by means of the asynchronous serial line. In particular, it can select the type of transducer used if the plate has transducers of several types (encoders or potentiometers or laser devices), it can configure the measurement-acquisition frequency and, finally, it can enable/ disable the interface.

The software allows the swaying of the plate transmitted by the user's foot, expressed in angular degrees relative to the horizontal, to be displayed in real time by means of a visual trace on the monitor (showed as a continuous line, horizontal bars or vertical columns), and to assess the equilibrium management ability of the subject with stances of various types (monopodal, bilateral, in lateral sway control and in forward-backward control) with bare feet or with footwear and also with footwear specific to a certain sports discipline. Lateral sway control is preferably shown graphically by horizontal bars about a vertical axis. Forward-backward control is preferably shown by vertical columns about a horizontal axis. The bars and the columns may be replaced by continuous lines. The fact that portions of the trace can be enlarged permits detailed analysis of the swaying of the rocking plate. The high sampling frequencies enable the trace to be compared with other forms of movement study (images from high-frequency video cameras, electroencephalographic and electromyographic traces, etc.).

In particular, the signal-processing devices are configured to generate some performance indices from the signal generated by the transducers, that is: the total area of deviation from the horizontal plane, areas and times for supination and pronation, or for dorsal and plantar flexion, or for inclination to the right or to the left, the ratio between the supination and pronation areas, the ratio between supination and pronation times, the area outside a certain selectable angle of inclination, etc.

The extent of supination and pronation (or of dorsal or plantar flexion, or of inclination to the right or to the left) of the test may be expressed rather than as areas, as average degrees of supination or pronation (or dorsal or plantar flexion, or of inclination to the right or to the left). The extent of overall deviation may be expressed as the sum of the average degrees of supination and pronation (or dorsal and plantar flexion, or of inclination to the right and to the left).

The software also allows the training and rehabilitation programs to be individualised:
a) showing on the monitor a "path" represented by a trace of a different colour with respect to the trace generated by the subject attempting to track, as close as possible to the selected "path";
b)using paths represented by pairs of straight or curved "guide lines" which can be positioned at will, and within which the person has to keep the trace produced by the swaying of the rocking plate.

For this purpose, the software is arranged for guiding the user by presenting on the monitor a graphic representation of a predetermined path of deviation from the equilibrium condition.

The paths may be shown as areas or as continuous lines and may be placed in the foreground or in the background relative to the real time test. Moreover, it is possible to construct a path with "giant slalom gates" which the person has to pass through with the real time trace. The possibility to display the various parts of the path to be performed and the gate to be passed through only a few moments before they have to be tackled ("anticipation" function) results in ever faster programming of the motor response. The "ZOOM-+" function permits both detailed analysis of micro-movements (by displaying them even when sampling takes place every 4ms) and an overall view of prolonged tests. The "ZOOM IN ACQUISITION" function, on the other hand, permits programmable variation of the trace on the monitor (that is, modification of the time axis) even during a test. It is thus possible to increase or reduce the density of tasks to be performed per unit of time. In accordance with the foregoing, the software therefore comprises a comparison function which can compare a datum of the actual path of deviation from the equilibrium condition by the user, derived from the signal generated by the transducer, with the predetermined path displayed, generating a corresponding deviation signal which can be displayed to the user.

Further options comprise: the "random" option, that is, the random selection by the personal computer of the "path" to be reproduced; the "count-down" which is activated for a certain period of time without warning, and the possibility to compare oneself with other subjects on the same paths.

By means of the "pace-maker" function, the monitor rather than showing the trace, only displays, one after the other, a sequence of bright dots along the central axis, as a function of the elapsed time (the rate of appearance of said dots, which is the expression of the progress of the invisible trace, may be constant or may vary not only among different tests, but also within the frame of a single test). The individual under test may therefore know only the time position of the trace with respect to assigned task (path to be reproduced), whereas he has no visual feed-back information about the space position of the rocking plate.
By the end of the test, the trace is displayed, which allows, together with the numerical data, to compare the test not only with others of the same type, but also with tests carried out in the presence of the visual feed-back. The exclusion of the space component of the visual feed-back allows in fact a further improvement of the system, by optimising the capacity of using the proprioceptive signals coming from the articular districts (particularly the distal districts) of the supporting limb or limbs and the quality of the corresponding neuromuscular response.

The "scrolling" function maintains the trace at the centre of the display, whereas, in this case, the path to be reproduced flows in the background.

The "virtual parallel" function allows to carry out a parallel test, by displaying, in real time on the same view, another test of the same or other individual performed beforehand.

The "average correction time" function evaluates the average time employed by the individual during the test, in order to correct the position of the rocking plate, whenever the degree of inclination has overcome a given value which may be defined by the user.

It is also possible, by means of the "training" function, for the subject, the trainer or the rehabilitator to program true training sessions establishing exercises sequences with different characteristics to be performed in succession with programmable recovery times between one test and another, thus modulating the engagement of the various muscle groups and of the central nervous system. After it has been started, the session proceeds automatically, presenting the sequence of predetermined exercises one after another. The training session may be set to pause at any time.

In the early stages of rehabilitation, the exercises may be performed seated or standing but with one foot resting on an inverted U-shaped structure which extends over the plate like a bridge, leaving the plate free to sway.

The high sensitivity of the system allows the individual suffering from neurological or neuromuscular problems to perceive even minor improvements which characterise rehabilitation in this field, thus allowing the rehabilitator to plan effective and progressive action in view of the relevant feed-back information which the interactive device provides to the patient.

By virtue of the characteristics set forth above, the device according to the invention is not only able to help in the recovery of the functionality of muscle-tendon-ligament and neuromuscular structures but represents an innovative instrument for training the equilibrium capacity and the disequilibrium management in healthy people.

Naturally, the principle of the invention remaining the same, the details of embodiment and forms of construction may be varied widely with respect to those described and illustrated by way of non-limiting example. Thus, for example, the device may provide for transducers of types other than those described.

The operation of the transducer which has been described with reference to a hardened-steel lamina fitted in a metal support fixed to the control may be varied and may thus be constituted, for example, by mechanical systems, by swinging sensors of other types, by push-buttons, electrical contacts, devices moved vertically, horizontally or with the rotation of gears, and systems with infra-red or laser beams or low- or high-frequency radio pulses and electromagnetic systems which can detect the swaying of the plate.

The microprocessor which constitutes the electrical interface connecting the plate to the personal computer may be mounted on board the rocking plate (i.e. on its lower surface facing the fixed support), or in an external housing.

The interface and the transducer may be supplied by means of a serial or parallel port, either by a specific card in the PC, or by a suitable external supply.

## Claims

1. A device with a balance plate, particularly for the assessment and training of the 'equilibrium capacity and disequilibrium management and for the evaluation, training and rehabilitation of the proprioceptive sensitivity and reflexes, comprising a plate (2) rocking about at least one axis of rotation and having an upper surface (4) for supporting the user's feet, the rocking plate (2) having at least one member (8, 10, 10b) for contacting a fixed support (16) and transducer means (26, 27) for converting the angular position of the plate relative to the horizontal into an electrical signal indicative of the user's reactions, **characterised in that** the contact member (8, 10, 10b) has a profile (12, 14, 14b), where the centre of the radius for all possible points of contact with the support (16) is always above the plate's upper surface (4) when in use, in contact with the horizontal and planar support (16) such that the instantaneous centre of rotation of the rocking plate is movable in space along a predetermined plane located above the upper support surface (4) in dependence on the instantaneous angular position of the rocking plate relative to the fixed support (16), whereby the contact member (8, 10, 10b) and the support form a rocking system with a movable fulcrum.

2. A device according to claim 1, **characterised in that** the contact member has a cylindrical surface (12, 14) for contact with the fixed support.

3. A device according to claim 1, **characterised in that** the contact member has a spherical surface (14b) for contact with the fixed support.

4. A device according to any one of claims 1 to 3, in which the contact surface of said contact member has a radius of curvature of between 4 and 10 cm.

5. A device according to any one of Claims 1 to 4, comprising a pair of spaced-apart contact members with circular cylindrical surfaces.

6. A device according to any one of the preceding Claims, in which the transducer is a potentiometer (26), an encoder (26) or an optical laser device (27).

7. A device according to Claim 6, **characterised in that** the transducer is a potentiometer (26) mounted firmly on the rocking plate.

8. A device according to any one of the preceding Claims, comprising, in combination, a fixed support (16) connected to the rocking plate by engagement means allowing the plate to sway.

9. A device according to Claim 8, **characterised in that** the engagement means comprise, for each of the contact members, a pin (18) inserted in a through-hole (20) in the contact member (12, 14) and slidable in an arcuate slot (22) formed in a flange (24) fixed firmly to the fixed support and perpendicular to the fixed support.

10. A device according to any one of the preceding Claims, comprising signal-processing means (34) configured for generating, from the transducer signal, at least one performance signal selected from one or more of the following:
- the total area of deviation from the horizontal plane, areas and/or times for supination and/or pronation, areas and/or times for dorsal and/or plantar flexion or for inclination to the right or to the left, the ratio between the supination and pronation areas, the ratio between the supination and pronation times, the area outside a certain selectable angle of inclination.

11. A device according to any one of the preceding Claims, further comprising means for the graphic display of the performance signal.

12. A device according to Claim 11, in which the processing means are arranged to implement a program for guiding the user by presenting on the display means a graphic representation of a predetermined path of deviation from the equilibrium condition.

13. A device according to Claim 11 or Claim 12, in which the processing means comprise a comparison function for comparing a datum of the actual deviation from the equilibrium condition by the user, derived from the signal supplied by the transducer, with a predetermined path displayed, and for generating a corresponding deviation signal which can be displayed to the user.

14. A device according to any one of the preceding Claims, in which the downward offset of the plate, meaning the distance of the support surface from the instantaneous centre of rotation, is between 0.5 and 9 cm.

## Patentansprüche

1. Vorrichtung mit einer Platte zum Halten des Gleichgewichts, im Besonderen zur Abschätzung und zum Training des Gleichgewichtsvermögens und der Behandlung von Gleichgewichtsstörungen sowie zur Beurteilung, zum Training und zur Wiederherstellung der propriozeptiven Empfindlichkeit und der propriozeptiven Reflexe, wobei die Vorrichtung eine Platte (2) enthält, die sich um zumindest eine Drehachse verschwenken kann und eine obere Fläche (4) besitzt, um die Füße eines Benützers zu tragen, wobei die Schwenkplatte (2) zumindest ein Element (8, 10, 10b) enthält, um mit einer ortsfeste Auflage (16) in Berührung zu treten, sowie eine Wandlereinrichtung (26, 27) enthält, um die Winkelstellung der Platte relativ zur Horizontalen in ein elektrisches Signal umzusetzen, das die Reaktionen des Benützers anzeigt, **dadurch gekennzeichnet, dass** das Berührungselement (8, 10, 10b) eine Mantelfläche (12, 14, 14b) besitzt, bei der der Mittelpunkt des Radius von allen möglichen Berührungspunkten mit der Auflage (16) immer oberhalb der oberen Fläche (4) der Platte liegt, wenn diese im Betrieb mit der horizontalen und planaren Auflage (16) in Berührung steht, so dass das Momentanzentrum der Schwenkplatte im Raum längs einer vorgegebenen Ebene, die oberhalb der oberen Tragfläche (4) angeordnet ist, in Abhängigkeit von der momentanen Winkelstellung der Schwenkplatte relativ zur ortsfesten Auflage (16) bewegbar ist, wodurch das Berührungselement (8, 10, 10b) und die Auflage ein Schwenksystem mit einem bewegbaren Drehpunkt bilden.

2. Vorrichtung gemäß Anspruch 1, **dadurch gekennzeichnet, dass** das Berührungselement eine zylindrische Fläche (12, 14) besitzt, um mit der ortsfesten Auflage in Berührung zu treten.

3. Vorrichtung gemäß Anspruch 1, **dadurch gekennzeichnet, dass** das Berührungselement eine sphärische Fläche (14b) besitzt, um mit der ortsfesten Auflage in Berührung zu treten.

4. Vorrichtung gemäß irgendeinem der Ansprüche 1 bis 3, wobei die Berührungsfläche des Berührungselements einen Krümmungsradius zwischen 4 und 10 cm besitzt.

5. Vorrichtung gemäß irgendeinem der Ansprüche 1 bis 4, wobei die Vorrichtung ein Paar von voneinander beabstandeten Berührungselementen mit kreiszylinderförmigen Flächen enthält.

6. Vorrichtung gemäß irgendeinem der bisherigen Ansprüche, wobei der Wandler aus einem Potentiometer (26), einem Kodierer (26) oder einer optischen Lasereinrichtung (27) besteht.

7. Vorrichtung gemäß Anspruch 6, **dadurch gekennzeichnet, dass** der Wandler aus einem Potentiometer (26) besteht, das an der Schwenkplatte fest angebracht ist.

8. Vorrichtung gemäß irgendeinem der bisherigen Ansprüche, wobei die Vorrichtung in Kombination eine ortsfeste Auflage (16) besitzt, die mit der Schwenkplatte über eine Eingreifeinrichtung verbunden ist, die ein Schaukeln der Platte ermöglicht.

9. Vorrichtung gemäß Anspruch 8, **dadurch gekennzeichnet, dass** die Eingreifeinrichtung für jedes der Berührungselemente einen Zapfen (18) enthält, der in eine Durchgangsöffnung (20) im Berührungselement (12, 14) eingesetzt wird und in einem bogenförmigen Schlitz (22) gleiten kann, der in einem Flansch (24) ausgebildet ist, der an der ortsfesten Auflage fest und senkrecht zur ortsfesten Auflage angebracht ist.

10. Vorrichtung gemäß irgendeinem der bisherigen Ansprüche, wobei die Vorrichtung eine Signalprozessorstufe (34) enthält, die so aufgebaut ist, um aus dem Wandlersignal zumindest ein Kennsignal zu erzeugen, das aus einem oder aus mehreren der folgenden Begriffe ausgewählt wird:
- aus dem gesamten Bereich der Abweichung von der Horizontalebene, aus den Bereichen und/oder Zeiten der Supination und/oder der Pronation, aus den Bereichen und/oder Zeiten für eine dorsale und/oder plantare Flexion oder für eine Neigung nach rechts oder nach links, aus dem Verhältnis zwischen den Supinations- und Pronationsbereichen, aus dem Verhältnis zwischen den Supinations- und Pronationszeiten, aus dem Bereich außerhalb eines bestimmten auswählbaren Neigungswinkels.

11. Vorrichtung gemäß irgendeinem der bisherigen Ansprüche, wobei die Vorrichtung weiters eine Einrichtung enthält, um das Kennsignal grafisch darzustellen.

12. Vorrichtung gemäß Anspruch 11, wobei die Prozessorstufe so aufgebaut ist, um ein Programm zu implementieren, um den Benützer dadurch zu führen, dass es auf der Anzeigeeinrichtung eine grafische Darstellung eines vorgegebenen Wegs der Abweichung vom Gleichgewichtszustand darstellt.

13. Vorrichtung gemäß Anspruch 11 oder Anspruch 12, wobei die Prozessorstufe eine Vergleichsfunktion enthält, um eine Bezugsgröße der tatsächlichen Abweichung vom Gleichgewichtszustand durch den Benützer, die mit jenem Signal hergeleitet wird, das vom Wandler stammt, mit einem vorgegebenen Weg zu vergleichen, der dargestellt wird, und um ein entsprechendes Abweichungssignal zu erzeugen, das dem Benützer dargestellt werden kann.

14. Vorrichtung gemäß irgendeinem der bisherigen Ansprüche, wobei ein nach unten Versetzen der Platte, das heißt des Abstands der Oberfläche der Tragplatte vom Momentanzentrum, zwischen 0,5 und 9 cm liegt.

## Revendications

1. Appareil avec plateau d'équilibrage, en particulier pour l'évaluation et l'apprentissage de la capacité d'équilibre et de la gestion du déséquilibre et pour l'évaluation, l'apprentissage et la réhabilitation des réflexes et de la sensibilité proprioceptifs, comprenant un plateau (2) balançant autour d'au moins un axe de rotation et ayant une surface supérieure (4) pour supporter les pieds de l'utilisateur, le plateau balançant (2) ayant au moins un élément (8, 10, 10b) pour entrer en contact avec un support fixe (16) et des moyens transducteurs (26, 27) pour convertir la position angulaire du plateau par rapport à l'horizontale en un signal électrique indiquant les réactions de l'utilisateur, **caractérisé en ce que** l'élément de contact (8, 10, 10b) a un profil (12, 14, 14b), dans lequel le centre du rayon pour tous les points possibles de contact avec le support (16) est toujours au-dessus de la surface supérieure (4) du plateau en cas d'utilisation, en contact avec le support horizontal et planaire (16) de façon à ce que le centre de rotation instantané du plateau balançant soit mobile dans l'espace le long d'un plan prédéterminé situé au-dessus de la surface de support supérieure (4) en dépendance sur la position angulaire instantanée du plateau balançant par rapport au support fixe (16), moyennant quoi l'élément de contact (8, 10, 10b) et le support forment un système balançant avec un point d'appui mobile.

2. Appareil selon la revendication 1, **caractérisé en ce que** l'élément de contact a une surface cylindrique (12, 14) pour entrer en contact avec le support fixe.

3. Appareil selon la revendication 1, **caractérisé en ce que** l'élément de contact a une surface sphérique (14b) pour entrer en contact avec le support fixe.

4. Appareil selon l'une quelconque des revendications 1 à 3, dans lequel la surface de contact dudit élément de contact a un rayon de courbure compris entre 4 et 10 cm.

5. Appareil selon l'une quelconque des revendications 1 à 4. comprenant une paire d'éléments de contact espacés avec des surfaces cylindriques circulaires.

6. Appareil selon l'une quelconque des revendications précédentes, dans lequel le transducteur est un potentiomètre (26), un codeur (26) ou un dispositif à laser optique (27).

7. Appareil selon la revendication 6, **caractérisé en ce que** le transducteur est un potentiomètre (26) fixé solidement sur le plateau balançant.

8. Appareil selon l'une quelconque des revendications précédentes, comprenant, en association, un support fixe (16) relié au plateau balançant par des moyens de mise en prise permettant au plateau de balancer.

9. Appareil selon la revendication 8, **caractérisé en ce que** les moyens de mise en prise comprennent, pour chacun des éléments de contact, une broche (18) insérée dans un trou traversant (20) dans l'élément de contact (12, 14) et coulissant dans une fente en forme d'arc (22) formée dans une bride (24) fixée solidement au support fixe et perpendiculaire au support fixe.

10. Appareil selon l'une quelconque des revendications précédentes, comprenant des moyens de traitement de signal (34) configurés pour générer, à partir du signal du transducteur, au moins un signal de performance sélectionné à partir de l'un ou plus des suivants :
- la zone totale de déviation par rapport au plan horizontal, les zones et/ou les moments de supination et/ou pronation, les zones et/ou les moments de flexion dorsale et/ou plantaire ou d'inclinaison vers la droite ou vers la gauche, le rapport entre les zones de supination et de pronation, le rapport entre les moments de supination et de pronation, la zone à l'extérieur d'un certain angle d'inclinaison sélectionnable.

11. Appareil selon l'une quelconque des revendications précédentes, comprenant en outre des moyens pour l'affichage graphique du signal de performance.

12. Appareil selon la revendication 11, dans lequel les moyens de traitement sont arrangés pour mettre en oeuvre un programme pour guider l'utilisateur en présentant sur les moyens d'affichage une représentation graphique d'un chemin prédéterminé de déviation à partir de la condition d'équilibre.

13. Appareil selon la revendication 11 ou la revendication 12, dans lequel les moyens de traitement comprennent une fonction de comparaison pour comparer une donnée de la déviation réelle par rapport à la condition d'équilibre par l'utilisateur, découlant du signal fourni par le transducteur, avec un chemin prédéterminé affiché, et pour générer un signal de déviation correspondant qui peut être affiché à l'utilisateur.

14. Appareil selon l'une quelconque des revendications précédentes, dans lequel le décalage vers le bas du plateau, c'est-à-dire la distance de la surface de support depuis le centre de rotation instantané, est compris entre 0,5 et 9 cm.
